# EUROPEAN PATENT APPLICATION

(11) **EP 2 757 376 A1**
(43) Date of publication of application: **23.07.2014**
(21) Application number: 12831729.4
(22) Date of filing: 14.09.2012
(51) Int. Cl.: G01N 33/574, C07K 14/47, C07K 16/18, C12N 15/09

(54) **MOLECULAR MARKER FOR EARLY INDENTIFICATION OF PLEURAL MESOTHELIOMA PATIENTS, AND EXPRESSION ANALYSIS METHOD FOR SAME**

(30) Priority: 15.09.2011 JP 2011202463
(71) Applicant: National University Corporation Nagoya University, Aichi 464-8601 (JP); Medical and Biological Laboratories Co., Ltd., Aichi 460-0008 (JP); Oncomics Co., Ltd., Aichi 464-0858 (JP)
(72) Inventor: YANAGISAWA, Kiyoshi, Nagoya-shi Aichi 464-8601 (JP); TAKAHASHI, Takashi, Nagoya-shi Aichi 464-8601 (JP); YOKOI, Kohei, Nagoya-shi Aichi 464-8601 (JP); HASEGAWA, Yoshinori, Nagoya-shi Aichi 464-8601 (JP); ONO, Ken-ichiro, Ina-shi Nagano 396-0002 (JP); YAGI, Kasumi, Ina-shi Nagano 396-0002 (JP); MASUDA, Hitomi, Ina-shi Nagano 396-0002 (JP); TAKEUCHI, Toshiyuki, Nagoya-shi Aichi 464-0858 (JP)
(74) Representative: Kador & Partner
(86) International application number: PCT/JP2012/005878
(87) International publication number: WO 2013/038696

(57) **Abstract**

To develop a simple, low-cost and highly reliable testing method for mesothelioma, and a kit used for said testing method.

The present invention provides a testing method for mesothelioma comprising a step in which the concentration of human periostin protein is measured in at least one type of sample from among samples of the blood or pleural fluid of a subject. The step in which the concentration of human periostin protein is measured may use an antibody directed against human periostin protein. The present invention further provides a kit for diagnosing mesothelioma, said kit including the antibody directed against human periostin protein. In one kit for diagnosing mesothelioma, the diagnosis of mesothelioma determines if a subject that may have mesothelioma has mesothelioma or is healthy. In another kit for diagnosing mesothelioma, the diagnosis of mesothelioma determines if a subject that may have mesothelioma has mesothelioma or has a respiratory illness other than mesothelioma.

## Description

### Technical Field

The present invention relates to a method for testing mesothelioma, and a kit for diagnosing mesothelioma. Specifically, the present invention relates to a method for testing mesothelioma, comprising a step of determining the concentration of human periostin in at least one type of samples of blood and pleural fluid, and a kit for diagnosing mesothelioma, comprising an antibody directed against a human periostin protein.

### Background Art

Mesothelioma has been known to be caused by inhalation of asbestos. Mesothelioma is classified into pleural mesothelioma, peritoneal mesothelioma and pericardial mesothelioma in accordance with the location where it develops. Mesothelioma most frequently occurs at pleura, and pleural mesothelioma accounts for 80 to 90% of the entirety of the mesothelioma episodes (Nonpatent Document 1). Benign pleural mesothelioma is referred to as fibrous mesothelioma, and more specifically, as a result of pneumonia, injury or the like followed by desmoplasia at the pleura, a localized mass is formed. To the contrary, malignant pleural mesothelioma is a tumor that develops from pleural mesothelium (i.e., double membranes covering the lung-side surface of pleura encapsulating the lung), and is a highly malignant tumor. For malignant pleural mesothelioma, treatments involving a combination of therapies of surgery, internal medicine and radiology have been carried out; however, malignant pleural mesothelioma has been still one of tumors accompanied by poor prognoses. Mesothelioma is characterized by a very long latency period, and a time period from the exposure to asbestos to the onset of mesothelioma is reportedly 11 to 54 years (median: 38.6 years). In Japan, because asbestos had been most frequently used from 1970's to 1990's, the number of mesothelioma patients has been predicted to be increasing until 2030's. The long latency period, along with the lack of a screening method useful for early detection, results in belated diagnosis of the disease condition. In advanced cases, the prognosis can be extremely unfavorable. In most of such cases, the disease states advance rapidly, leading to a death within several years. However, in cases of early detection which provides an opportunity for curative surgical resection, one can expect a long term survival. Accordingly, development of a novel screening method that allows early detection has been an urgent issue.

Early diagnoses of malignant pleural mesothelioma are extremely difficult, and the disease state already falls under an advanced stage in most cases when malignant pleural mesothelioma was found due to subjective symptoms such as chest pain, and respiratory distress resulting from pleural effusion. Examples of a marker for use in diagnoses of malignant pleural mesothelioma are comprised of hyaluronic acid, CYFRA, CEA and the like in the pleural fluid; however, these involve significant problems such as necessities for collecting the pleural fluid as a sample, and inferior accuracy of the diagnosis. Moreover, in order to make a reliable diagnosis, it is necessary to carry out a precise histopathological diagnosis using a biopsy specimen, and thus an extremely heavy psychological, physical and social burden is imposed. In addition, malignant pleural mesothelioma is classified into epithelial type, sarcomatoid type, and mixed type of both (biphasic), based on histological types. Of them, prognosis of the sarcomatoid malignant pleural mesothelioma is extremely poor, without any effective diagnostic method. Therefore, when malignant mesothelioma is diagnosed, in many cases, it'has already been widely spread, and it is often impossible to carry out a radical surgery. Accordingly, prognoses are extremely poor, with one and two year survival rate of about 50% and about 20%, respectively.

Since non-neoplastic inflammatory lesion develops at lung and pleura as a result of exposure to asbestos, abnormal findings on X-ray have already been noticed in those exposed to asbestos even before developing mesothelioma. Therefore, X-ray examination has been considered to less useful as a means for screening. In addition, cases with mesothelioma are much fewer than those who have been exposed to asbestos. A simple, low-cost and highly reliable testing method for those exposed to asbestos has been strongly needed.

First, the present inventors focused attention on a fact that mesothelin is positive as a tumor marker in the blood only for epithelial mesothelioma, and attempted to search for a marker capable of detecting both epithelial and sarcomatoid pleural mesothelioma. For diagnoses of sarcomatoid pleural mesothelioma, cytological examination, pleura biopsy and image diagnosis have been proposed to be needed, and thus the diagnosis thereof has been extremely difficult (Nonpatent Document 2). The present inventors attempted and succeeded to obtain information on expression of several hundred proteins by analyzing protein expression profiles of pleural mesothelioma tissue samples to apply a mining technique of proteome analysis results with procedures such as IDA (Information Dependent Analysis) using a mass spectrometry instrument, a bioinformatics analysis making full use of databases, comparison with gene expression using DNA microarrays, prediction of a protein function by searching motif sequences, and quantitative determination by comparison with an internal standard sample.

Furthermore, in order to further facilitate the study for narrowing candidates as a marker in the blood, proteomics analyses on the blood, MRM (Multiple Reaction Monitoring), by using a mass spectrometry instrument was carried out. In addition, with regard to tens of proteins in particular, among the several hundred proteins described above, it was confirmed that the amount of proteins present in malignant pleural mesothelioma tissue samples is increased as compared with that of control normal pleura mesothelium tissue samples. Additionally, in blood samples from malignant pleural mesothelioma cases, it was confirmed that the concentration of periostin increased as compared with control cases of respiratory organ diseases other than mesothelioma.

Periostin is originally referred to as osteoblast specific factor-2 (OSF-2), and is a molecule separated and identified as a gene specifically expressed in mouse osteoblast strain MC3T3-E1 cells (Nonpatent Document 3). The amino acid sequence encoded by a periostin gene is comprised of a general signal sequence, a cysteine rich domain, four repeats of a Fasciclin 1 domain consisting of about 150 amino acid residues, and a carboxyl terminal domain. Periostin has four isoforms of splicing variants, in which only the carboxyl terminal region varies. With regard to periostin, it has been reported that high expression is found not in lung cancer parenchymal cells themselves but in stroma cells on the periphery of the parenchymal cells (Nonpatent Documents 4 and 5); that, although serum periostin is not useful in diagnosing the presence of non-small cell lung cancer, it would be promising as a marker for prognosis (Nonpatent Documents 4 and 5); that high expression is found in breast cancer (Nonpatent Document 6); that high expression is found in thymus cancer (Nonpatent Document 7); and that high expression is found in pancreatic cancer (Nonpatent Document 8). However, these cancers are completely different from mesothelioma in terms of disease conditions. Moreover, there are patent applications disclosing that an antibody recognizing amino acid residues of seventeenth exon of a carboxyl terminal domain which forms a splicing variant of periostin is effective in diagnoses and/or treatments of cardiac diseases and carcinomas other than lung cancer (Patent Documents 1 and 2). However, a relationship of mesothelioma with periostin expression and the presence in blood has not been known so far.

### Prior Art Documents

### Patent Documents

Patent Document 1: PCT International Publication No. WO2007/077934, pamphlet
Patent Document 2: PCT International Publication No. WO2009/001940, pamphlet

### Nonpatent Documents

Nonpatent Document 1: Fujimoto N. et al, J. Cancer Res. Clin. Oncol., 136: 1755, 2010
Nonpatent Document 2: Husain et al., Arch. Pathol. Lab. Med., 133: 1317, 2009
Nonpatent Document 3: Takeshita S. et al., Biochem. J., 294: 271, 1993
Nonpatent Document 4: Sasaki H. et al, Jpn. J. Cancer Res., 92: 869, 2001
Nonpatent Document 5: Sasaki H. et al, Cancer., 92: 843, 2001
Nonpatent Document 6: Shao R. et al, Mol. Cell Biol., 24: 3992, 2004
Nonpatent Document 7: Sasaki H. et al, Cancer Lett., 172: 37, 2001
Nonpatent Document 8: Baril P. et al, Oncogene, 26: 2082, 2007

### SUMMARY OF THE INVENTION

### Problems to be Solved by the Invention

There have existed needs for development of a simple, low-cost and highly reliable method for testing mesothelioma and a kit for use in the method for testing.

### Means for Solving the Problems

An aspect of the present invention provides a method for testing mesothelioma, the method comprising a step of determining the concentration of a human periostin protein in at least one type of samples of blood and pleural fluid of a subject.

In the method for testing mesothelioma according to the aspect of the present invention, an antibody directed against a human periostin protein may be used in the step of determining the concentration of the human periostin protein.

In the method for testing mesothelioma according to the aspect of the present invention, the antibody directed against a human periostin protein may be an antibody that binds to a polypeptide consisting of an amino acid sequence set out in SEQ ID NO: 2.

In the method for testing mesothelioma according to the aspect of the present invention, two types of antibodies capable of concurrently binding to the human periostin protein may be used.

In the method for testing mesothelioma according to the aspect of the present invention, the mesothelioma may be malignant pleural mesothelioma.

In the method for testing mesothelioma according to the aspect of the present invention, the mesothelioma may be sarcomatoid malignant pleural mesothelioma or biphasic malignant pleural mesothelioma.

In the method for testing mesothelioma according to the aspect of the present invention, the blood sample may be a plasma sample or a serum sample of the subject.

Another aspect of the present invention provides a kit for diagnosing mesothelioma, the kit comprising an antibody directed against a human periostin protein.

In the kit for diagnosing mesothelioma according to the aspect of the present invention, the antibody directed against a human periostin protein may be an antibody that binds to a polypeptide consisting of an amino acid sequence set out in SEQ ID NO: 2. In the kit, the concentration of the human periostin protein may be measured by an ELISA method.

In the kit for diagnosing mesothelioma according to the aspect of the present invention, the antibody directed against a human periostin protein may be two types of antibodies capable of concurrently binding to the human periostin protein.

The kit for diagnosing mesothelioma according to the aspect of the present invention may comprise a solid support on which one of the two types of antibodies capable of concurrently binding to the human periostin protein is immobilized, and a specific binding partner for another of the two types of antibodies. According to the kit, the concentration of the human periostin protein may be determined by a sandwich ELISA method.

In the kit for diagnosing mesothelioma according to the aspect of the present invention, diagnosing mesothelioma may determine whether or not a subject who is in fear of suffering from mesothelioma is in fact a patient suffering from mesothelioma.

In the kit for diagnosing mesothelioma according to the aspect of the present invention, diagnosing mesothelioma may determine whether a subject who is in fear of suffering from mesothelioma is in fact a patient suffering from mesothelioma or a respiratory organ disease other than mesothelioma.

In the kit for diagnosing mesothelioma according to the aspect of the present invention, the patient suffering from mesothelioma may be a patient suffering from malignant pleural mesothelioma.

In the kit for diagnosing mesothelioma according to the aspect of the present invention, the patient suffering from mesothelioma may be a patient suffering from malignant pleural mesothelioma or a patient suffering from biphasic malignant pleural mesothelioma.

Still another aspect of the present invention provides a method for diagnosing mesothelioma, the method comprising a step of determining the concentration of a human periostin protein in at least one type of samples of blood and pleural fluid of a subject.

In the method for diagnosing mesothelioma according to the aspect of the present invention, an antibody directed against a human periostin protein may be used in the step of determining the concentration of the human periostin protein.

In the method for diagnosing mesothelioma according to the aspect of the present invention, the antibody directed against a human periostin protein may be an antibody that binds to a polypeptide consisting of an amino acid sequence set out in SEQ ID NO: 2.

In the method for diagnosing mesothelioma according to the aspect of the present invention, two types of antibodies capable of concurrently binding to the human periostin protein may be used in the step of determining the concentration of the human periostin protein.

In the method for diagnosing mesothelioma according to the aspect of the present invention, diagnosing mesothelioma may determine as to whether or not a subject who is in fear of suffering from mesothelioma is in fact a patient suffering from mesothelioma.

In the method for diagnosing mesothelioma according to the aspect of the present invention, diagnosing mesothelioma may determine as to whether a subject who is in fear of suffering from mesothelioma is in fact a patient suffering from mesothelioma or a respiratory organ disease other than mesothelioma.

In the method for diagnosing mesothelioma according to the aspect of the present invention, the patient suffering from mesothelioma may be a patient suffering from malignant pleural mesothelioma.

In the method for diagnosing mesothelioma according to the aspect of the present invention, the patient suffering from mesothelioma may be a patient suffering from sarcomatoid malignant pleural mesothelioma or biphasic malignant pleural mesothelioma.

In the aspects of the present invention, mesothelioma is a tumor derived from a mesothelial cell, and may be comprised of any one of pleural mesothelioma, peritoneal mesothelioma and pericardial mesothelioma in accordance with the classification based on its location of original lesion. Also, malignant mesothelioma may be any one of epithelial type, sarcomatoid type, and mixed type (biphasic) thereof, according to the classification based on the cell type that constitutes the tumor parenchymal cells. Mesothelioma according to the aspects of the present invention may be pleural mesothelioma, and may be sarcomatoid malignant pleural mesothelioma. Since testing of mesothelioma is important for healthy individuals who have been exposed to asbestos, the method for testing mesothelioma, kit and/or method for diagnosing mesothelioma according to the aspects of the present invention are/is preferably applied to malignant pleural mesothelioma. In the method for testing mesothelioma by way of a conventional biochemical marker, it is extremely difficult to detect sarcomatoid malignant pleural mesothelioma and biphasic malignant pleural mesothelioma. To the contrary, the method for testing mesothelioma, kit and/or method for diagnosing mesothelioma according to the aspects of the present invention, can detect sarcomatoid malignant pleural mesothelioma and biphasic malignant pleural mesothelioma. Thus, the method for testing mesothelioma, kit and/or method for diagnosing mesothelioma according to the aspects of the present invention are/is preferably applied to sarcomatoid malignant pleural mesothelioma or biphasic malignant pleural mesothelioma. The method for testing mesothelioma, kit and/or method for diagnosing mesothelioma according to the aspects of the present invention may be used in order to distinguish a patient suffering from mesothelioma from a healthy individual. The method for testing mesothelioma, kit and/or method for diagnosing mesothelioma according to the aspects of the present invention may be used in order to distinguish a patient suffering from malignant pleural mesothelioma from a patient suffering from a respiratory organ disease other than malignant pleural mesothelioma. The respiratory organ disease other than malignant pleural mesothelioma as referred to herein may be comprised of lung cancer, and respiratory organ diseases other than cancer. The respiratory organ diseases other than cancer are comprised of, but not limited to, COPD and benign pleural mesothelioma.

The subject in the method for testing mesothelioma, kit and/or method for diagnosing according to the aspects of the present invention may be comprised of all humans who are required to take a test for mesothelioma. The subject in the method for testing mesothelioma according to the aspect of the present invention may exhibit symptoms such as cough, sputum, chest pain and respiratory distress. The subject may be a subject who may be suffering from mesothelioma. The subject who may be suffering from mesothelioma is comprised of, but not limited thereto, those who exhibit the symptoms, and/or those who have, or are suspected of having, inhaled or been exposed to asbestos.

The blood sample in the method for testing mesothelioma, kit and/or method for diagnosing mesothelioma according to the aspects of the present invention may be any one of whole blood, plasma and serum. The blood sample is preferably the plasma or serum separated after collecting the blood. Collection of the blood, and as needed, preparation of the plasma or serum, in the method for testing mesothelioma, kit and/or method for diagnosing mesothelioma according to the aspects of the present invention is carried out by using any of techniques well-known to persons skilled in the art. In brief, for the preparation of the plasma, cellular components are removed by centrifugation after adding a blood coagulation inhibitor comprising, but not limited to, a chelating agent such as EDTA or the like to the collected whole blood sample. For the preparation of the serum, a blood coagulation reaction is allowed to proceed in the collected whole blood sample, and then the serum is separated from the coagulated blood by centrifugal separation. Collection of the pleural fluid, and as needed, preparation of the pleural fluid specimen for the method for testing mesothelioma, kit and/or method for diagnosing mesothelioma according to the aspects of the present invention are carried out by using any of techniques well-known to persons skilled in the art (for example, see Porcel J. M. and Light R. W. (Am. Fam. Physician., 73: 1211, 2006)).

The human periostin protein in the method for testing mesothelioma, kit and/or method for diagnosing mesothelioma according to the aspects of the present invention means any human periostin gene product that is comprised of an amino acid sequence consisting of 630 amino acid residues listed in SEQ ID NO: 2, and a gene product of any of mutants of a human periostin gene loci that binds to an antibody that recognizes a polypeptide consisting of the amino acid sequence set out in SEQ ID NO: 2.

In the method for testing mesothelioma, kit and/or method for diagnosing mesothelioma according to the aspects of the present invention, the concentration of a human periostin protein may be measured by any method. In general, the concentration of the human periostin protein is determined by using any molecule capable of specifically binding to the human periostin protein. The concentration of the human periostin protein is preferably determined using an antibody that specifically binds to the human periostin protein. Here, the antibody that specifically binds to the human periostin protein may be selected from the group consisting of a polyclonal antibody or a monoclonal antibody, an antigen-binding fragment of the antibody, and a recombinant antibody or a chimeric antibody containing the antigen-binding fragment.

The polyclonal antibody or monoclonal antibody that specifically binds to the human periostin protein is produced using any of techniques well-known to persons skilled in the art. Although Examples herein disclose a technique for producing a monoclonal antibody that specifically binds to the human periostin protein, any antibody may be used in the method for testing mesothelioma, kit and/or method for diagnosing mesothelioma according to the aspects of the present invention, without limitation to the monoclonal antibody described in Examples.

The "antigen-binding fragment of the antibody" as referred to herein means a part of the antibody participating in antigen binding. The antigen-binding site is formed from amino acid residues of variable (V) regions of N-termini of a heavy (H) chain and a light (L) chain. The antigen-binding fragment is comprised of an Fab fragment or an F(ab')₂ fragment obtained by proteolysis of an intact polyclonal antibody or monoclonal antibody with proteolytic enzyme papain or pepsin, respectively, as well as an Fv fragment comprising a hetero dimer of non-covalently bound V_{H} and V_{L} regions that is comprised of the antigen-binding site carrying most of an antigen recognizing capacity and binding capacity of a natural antibody molecule.

With regard to the antibody according to the aspects of the present invention, the recombinant antibody may be prepared by expression cloning of an antibody gene involving either transformation into an appropriate bacterial host, or transfection into an appropriate mammalian cell host. With regard to the antibody according to the aspects of the present invention, the chimeric antibody may be a fusion protein supported by a constant domain of a homologous or heterogeneous antibody such that the antigen-binding site of the recombinant antibody of the aspects of the present invention can bind to the polypeptide described above. The chimeric antibody is comprised of a single chain variable region antibody (scFv) containing an antibody heavy chain variable region (V_{H}) operably linked to an antibody light chain variable region (V_{L}), and a camel heavy chain antibody (HCAb) that is of an IgG class free from a light chain produced by a camelid animal (Camelidae; including camel, Arabian camel and lama) or a heavy chain variable domain (V_{H}D) thereof. The recombinant antibody can be prepared in large quantities using an expression system of a gene derived from a prokaryote or a eukaryote.

The "step of determining the concentration of a human periostin protein in at least one type of samples of blood and pleural fluid of a subject" in the method for testing mesothelioma, kit and/or method for diagnosing according to the aspects of the present invention may be carried out by detection or quantitative determination by way of labelling with an anti-periostin antibody that binds to periostin in at least one type of samples of the blood and pleural fluid of the subject, a specific binding partner for the anti-periostin antibody, or the like. The anti-periostin antibody may be labelled directly or via a linker, with a low molecular ligand such as biotin, an enzyme, a radioisotope, a dye, a fluorescent dye, a chemiluminescent compound, or the like. The specific binding partner for the anti-periostin antibody is comprised of any substances capable of specifically binding to the anti-periostin antibody, which are known to persons skilled in the art. The specific binding partner for the anti-periostin antibody may be any receptor or ligand capable of binding to the anti-periostin antibody provided that the measuring of the concentration of a periostin protein in at least one type of samples of the blood and pleural fluid of the subject is not hampered. The receptor or ligand may be a protein, a carbohydrate, a nucleic acid, a lipid, or other biopolymer. The specific binding partner for the anti-periostin antibody used in the aspects of the present invention may be selected from the group consisting of a polyclonal antibody or a monoclonal antibody, an antigen-binding fragment of the antibody, a chimeric antibody or a recombinant antibody comprising the antigen-binding fragment. The antigen-binding fragment of the antibody is defined as a part of the antibody participating in antigen binding. The antigen-binding site is formed by amino acid residues of N-terminal variable (V) regions of a heavy (H) chain and a light (L) chain. The specific binding partner for the anti-periostin antibody may be labelled with an enzyme, a radioisotope, dye, a fluorescent dye or the like. The enzyme is peroxidase (POD), beta-galactosidase (β-Gal), alkali phosphatase (ALP) or the like. The enzyme is generally used in combination with an appropriate substrate. The step of detecting may be comprised of a step of measuring the absorbance with a spectrophotometer, or a step of measuring the luminescent or fluorescent intensity with a luminescence or fluorescence measuring device. In addition, latex particles or other fine particles may be bound. In such a case, the step of detecting may be comprised of a step of measuring the turbidity using a nephelometer. The step of quantitatively determining may be comprised of a step of producing a calibration curve by quantitating and plotting the amount of the anti-periostin antibody that binds to a known concentration or amount of a human periostin protein, and a step of determining the concentration or amount of the human periostin protein in the blood sample of a subject from the amount of the anti-periostin antibody that bound to the human periostin in an unknown concentration or amount of at least one type of samples of the blood and pleural fluid protein of the subject, using the calibration curve. In order to produce the calibration curve, at least two types of human periostin proteins with a known concentration or amount may be provided, and it is preferred that three types or four types or more of human periostin proteins are provided in order to ensure the accuracy for the quantitative determination. The step of detecting or determining quantitatively may be comprised of a step of eliminating the anti-periostin antibody and other antibody that failed to bind to the subject sample by washing. In this regard, a measuring technique carried out with an enzyme-labeled antibody or the like is referred to as an ELISA method. Also, a measuring technique in which two types of anti-human periostin protein antibodies capable of concurrently binding to a human periostin protein are used in order to capture the human periostin protein in the sample onto a solid support, and to detect or quantitatively determine the human periostin protein captured onto the solid support is referred to as a sandwich ELISA method. The two types of anti-human periostin antibodies may be either two types of monoclonal antibodies having different epitopes, or a combination of a monoclonal antibody and a polyclonal antibody.

When the concentration of a human periostin protein in at least one type of samples of the blood and pleural fluid of a subject is determined in the diagnostic method for mesothelioma according to the aspects of the present invention, the subject is determined to be susceptible to mesothelioma in the case in which the concentration is greater than a reference value. The reference value may be calculated by a statistical work that is well-known to persons skilled in the art.

The "step of measuring the concentration of a human periostin protein in at least one type of samples of the blood and pleural fluid of a subject" in the method for testing mesothelioma, kit and/or method for diagnosing according to the aspects of the present invention may be carried out by a variety of procedures known to persons skilled in the art. For example, the procedure may be conducted by an ELISA method explained hereinabove, and a latex aggregation method, as well as a surface plasmon resonance method.

The kit for executing the immunological analysis method according to the aspect of the present invention may involve the anti-periostin antibody of the aspect of the present invention, as well as a known concentration or amount of a control sample for producing a calibration curve. The control sample may be a human periostin protein, or a fusion protein thereof.

Yet another aspect of the present invention provides an immunological analysis apparatus of periostin configured such that the method for diagnosing mesothelioma according to the aspect of the present invention can be carried out. The apparatus is comprised of a detection unit such as a CCD camera, a measuring unit such as a spectrophotometer, a fluorescent spectrophotometer, a nephelometer and a surface plasmon resonance measuring apparatus, dispenser units for infusing and/or removing reagents, washing liquids and/or samples, a robot arm unit for handling multiwell plates for an ELISA method and sensor chip for surface plasmon resonance, a control unit for controlling, and the like.

All references cited herein are hereby incorporated by reference in their entirety.

### BRIEF DESCRIPTION OF THE DRAWINGS

Fig. 1 shows a schematic view illustrating relation among positions of functional domains of a human periostin protein, the structure of each isoform, and the structure of the immunogen used for producing the periostin antibody of the embodiment of the present invention.
Fig. 2A shows a graph demonstrating results of an examination on reactivity of monoclonal antibodies produced using a human periostin recombinant protein POSTN781 as an immunogen by using a microplate coated with POSTN781.
Fig. 2B shows a graph demonstrating results of an examination on reactivity of monoclonal antibodies produced using a human periostin recombinant protein POSTN630 as an immunogen by using a microplate coated with POSTN630.
Fig. 3 shows a graph illustrating distributions of concentrations of periostin in plasma specimens from mesothelioma patients and healthy individuals, respectively.
Fig. 4 shows a correlation diagram obtained by plotting concentrations of periostin and SMRP in plasma from the identical specimen, for plasma specimens from mesothelioma patients.
Fig. 5 shows a graph demonstrating comparison between concentrations of periostin in a plasma specimen and a serum specimen among specimens from a patient.
Fig. 6 shows a graph demonstrating comparison between distributions of concentrations of periostin in specimens from patients of malignant pleural mesothelioma of an Embodiment of the present invention, lung cancer, and a respiratory organ disease other than cancer (represented by "respiratory organ disease" in Fig. 6).
Fig. 7 shows a graph illustrating an ROC curve drawn using the concentrations of periostin as a marker, of specimens from malignant pleural mesothelioma patients of an Embodiment of the present invention with respect to patients suffering from respiratory organ diseases other than mesothelioma, including lung cancer and a respiratory organ disease other than cancer in combination.
Fig. 8 shows a graph demonstrating results obtained by measuring periostin in culture supernatants of established cancer cells.

### Description of Embodiments

Examples of the invention described below have been presented for the purpose of illustration only, and are not to be construed as limiting the technical scope of the invention. The technical scope of the present invention is limited solely by the language of the appended claims. Modifications of the present invention such as, for example, additions, deletions and substitutions of features of the present invention may be made without departing from the spirit of the present invention.

### Example 1

### Production of Anti-periostin Antibody

### (1) Structure of Human Periostin Protein

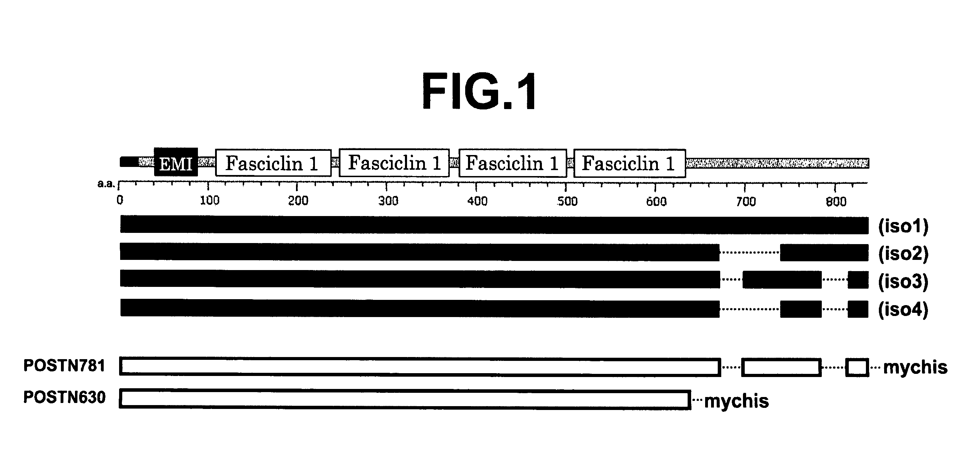

Fig. 1 shows a schematic view illustrating relation among positions of functional domains of a human periostin protein, the structure of each isoform, and the structure of the immunogen used for producing the periostin antibody of the present invention. In Fig. 1, EMI and Fasciclin 1 represent an EMILIN homologous domain and a fasciclin homologous domain, respectively. "iso1" to "iso4" represent isoforms 1 to 4 of human periostin, respectively. POSTN781 represents a recombinant protein obtained by fusing myc tag and his tag (represented by "mychis") to a carboxyl terminal of a polypeptide having 781 amino acid residues of a human periostin protein isoform 3. POSTN630 represents a recombinant protein obtained by fusing myc tag and his tag to a carboxyl terminal of a polypeptide having 630 amino acid residues that are common to all isoforms of the human periostin protein covering four fasciclin domains. The amino acid sequence having 781 amino acid residues of the human periostin protein isoform 3 is listed in SEQ ID NO: 1. The amino acid sequence of 630 amino acid residues covering four fasciclin domains that are common to all isoforms of the human periostin protein is listed in SEQ ID NO: 2.

### (2) Acquisition of Human Periostin cDNA

Based on cDNA sequences of four types of isoforms of the human periostin protein (isoform 1: NM_006475, isoform 2: NM_001135934, isoform 3: NM_001135935, and isoform 4: NM_001135936), primers were designed for a 5' UTR region and a 3' UTR region that are common to isoforms 1 to 4. The primers used for a PCR reaction of the first stage were 5'-AATTCTGAGCTCTCCAAAGCCC-3' (SEQ ID NO: 3) and 5'-GGCTAACTCCACAATTTCCCTC-3' (SEQ ID NO: 4), and the primers used for a PCR reaction of the second stage were 5'-CGGAGAGACTCAAGATGATTCC-3' (SEQ ID NO: 5) and 5'-TCCTGAAGTCAACTTGGCTCTC-3' (SEQ ID NO: 6). Using a mixture of human cDNA library inserts (mosaic cDNA (trademark), Genofi, LLC, San Clemente, CA 92673) as a template, a cDNA containing a full length protein-coding region of periostin was amplified by nested PCR using an enzyme for PCR amplification (KOD FX, Toyobo Co., Ltd.) and the primers. The first-stage PCR reaction was carried out under a condition involving 25 cycles of: at 98°C for 20 sec; at 55°C for 20 sec; and at 68°C for 2 min and 30 sec, and the second-stage PCR reaction was carried out under a condition involving 30 cycles of: at 98°C for 15 sec; at 55°C for 15 sec; and at 68°C for 2 min and 30 sec. The amplification product of the second-stage PCR reaction was cloned into a cloning vector, pT7Blue T-Vector (Novagen, Merck & Co., Inc.,), and the base sequence was confirmed using an automatic sequencer (Applied Biosystems, Inc.). Since the cloned cDNA agreed with the isoform 3, it was designated as POSTNiso3-pT7.

### (3) Construction of Secretory Expression System of Human Periostin Recombinant Protein

As a vector for allowing a human periostin recombinant protein to be expressed in animal cells, pQCxmhIPG was used which is controlled under a CMV promoter, and which is capable of co-expressing a Puromycin-EGFP fusion protein and a target gene product by an IRES sequence. pQCxmhIPG was produced by the inventors through modifying a pQCXIP vector of BD Retro-X (trademark) Q Vectors. POSTN781 expression vector was constructed as follows. Amplification of the target sequence by PCR was carried out using KOD FX, with POSTNiso3-pT7 as a template. A 5' primer (5'-CGGGCGGCCGCACCATGATTCCCTTTTTAC-3', SEQ ID NO: 7, underlined part: NotI recognition sequence), and a 3' primer (5'-TTTTTGGACTCGAGCTGAGAACGACCTTCC-3', SEQ ID NO: 8, underlined part: XhoI recognition sequence) were employed. The reaction condition involved 30 cycles of: at 94°C for 15 sec; at 55°C for 15 sec; and at 68°C for 2 min and 30 sec. The PCR reaction product thus obtained was digested with restriction enzymes NotI and XhoI, and inserted into a NotI-XhoI site of pQCxmhIPG to construct an expression vector pQCxmhIPG-POSTN781. A POSTN630 expression vector was constructed as follows. Amplification of the target sequence by PCR was carried out using the POSTNiso3-pT7 as a template, with a Pfu enzyme (Promega Corporation). A 5' primer (5'-CGGGCGGCCGCACCATGATTCCCTTTTTAC-3', SEQ ID NO: 9, underlined part: NotI recognition sequence) and a 3' primer (5'-GGTGTCTCGAGTGGATAGAGGAGTTTATC-3', SEQ ID NO: 10, underlined part: XhoI recognition sequence) were employed. The reaction condition involved 30 cycles of: at 98°C for 15 sec; at 55°C for 15 sec; and at 68°C for 2 min and 30 sec. The PCR reaction product thus obtained was digested with restriction enzymes NotI and XhoI, and inserted into a NotI-XhoI site of pQCxmhIPG to construct an expression vector pQCxmhIPG-POSTN630.

In order to establish a secretory expression cell strain of a human periostin recombinant protein, a retroviral packaging cell system (Pantropic Retroviral Expression System, Clontech, K1063-1, Takara Bio Inc.) was used. On a 100 mm dish coated with collagen, 80 to 90% confluent GP2-293 (Clontech, K1063-1, Takara Bio Inc.) was provided, and the expression vector pQCxmhIPG-POSTN781 or pQCxmhIPG-POSTN630, and pVSV-G (Clontech; K1063-1) each in an amount of 11.2 µg were co-transfected using Lipofectamine 2000. Forty eight hours later, the supernatant containing viral particles was recovered, and the viral particles were separated as precipitates by ultracentrifugation (at 18, 000 rpm, for 1.5 hours at 4°C). The precipitates were suspended in 30 µL of TNE (50 mM Tris-HCl (pH: 7.8), 130 mM NaCl and 1 mM EDTA) to prepare a concentrated liquid of a retroviral vector. Five µL of the concentrated liquid of the retroviral vector was diluted with 150 µL of DMEM (D5796, Sigma-Aldrich Japan K.K.) containing 8 µg/mL hexadimethrine bromide (H-9268, Sigma-Aldrich Japan K.K.) and 10% bovine serum to prepare a viral particle-containing medium. A 293T medium prepared to give about 40% confluence in a 96-well microplate was replaced with the viral particle-containing medium, whereby the pQCxmhIPG-POSTN781 or pQCxmhIPG-POSTN630 gene was introduced. After introducing the gene, extended culture was carried out in DMEM containing 5 µg/mL Puromycin (P-8833, Sigma-Aldrich Japan K.K.) and 10% fetal bovine serum to establish expression cell strains POSTN781/st293T and POSTN630/st293T of the human periostin recombinant protein.

### (4) Purification of Human Periostin Protein

The expression cell strains POSTN781/st293T and POSTN630/st293T of the human periostin recombinant protein were each subjected to 1 L culture in a serum free medium CD293 for 293 cells (Invitrogen, Life Technologies Japan Ltd.). The recombinant proteins POSTN781 and POSTN630 were recovered from the culture supernatant using TALON Purification Kit (Clontech, K1253-1), and dialyzed against PBS. Purified proteins were verified by SDS-PAGE and Western blotting. The protein concentration was determined using a protein assay kit II (BioRad, 500-0002JA, Bio-Rad Laboratories, Inc.).

### (5) Production of Monoclonal Antibody against Human Periostin Recombinant Protein

The human periostin recombinant proteins POSTN781 and POSTN630 purified as described in the section (4) were emulsified by mixing with the same amount of a complete adjuvant (F5881, Sigma-Aldrich Japan K.K.), and 5 to 20 µg thereof per animal was used for sensitization of BALB/c mouse (4 weeks old, female) every 3 to 7 days, six times in total. After 3 days following final immunization, lymphocyte cells were removed from the mouse, and cell fusion with mouse myeloma cells P3U1 (P3-X63Ag8U1) was carried out.

Cell fusion was carried out as follows in accordance with a routine method. Fetal bovine serum in all media was inactivated by a treatment of incubation at 56°C for 30 min. P3U1 was cultured in RPMI1640 medium supplemented with penicillin, streptomycin and 10% fetal bovine serum. The removed mouse lymphocyte cells and P3U1 were mixed at a ratio of 1:10 to 1:2, and the mixture was centrifuged. Thus precipitated cells were gently mixed while gradually adding 50% polyethylene glycol 4000 (1.09727.0100, Merck & Co., Inc.), and thereafter the mixture was centrifuged. The precipitated fusion cells were appropriately diluted with a HAT medium (RPMI1640, HAT-supplement (Invitrogen, 11067-030, Life Technologies Japan Ltd.), penicillin and streptomycin) containing 15% FBS, and seeded onto a 96-well microplate in a volume of 200 µL per well. The fusion cells were cultured in a CO₂ incubator (5% CO₂, 37°C), and when colonies were formed, sampling followed by screening of the culture supernatant was carried out. According to the screening, fusion cell colonies in wells that exhibited positive results by the ELISA method on the 96-well plate coated with POSTN781 or POSTN630 employed for immunization were selected. After extended culture was carried out in an HT medium (RPMI1640, HT-supplement (Invitrogen, 21060-017), penicillin and streptomycin) containing 15% fetal bovine serum, cloning was carried out by a limiting dilution method. Accordingly, 13 hybridoma clones (antibody Nos: #3-4-5, #3-6-1, #3-9-2, #3-10-2, #3-11-4, #3-20-3, #3-27-1, #3-28-3, #3-29-1, #3-30-1, #3-31-3, #3-33-3 and #3-34-1) were obtained with the human periostin recombinant protein POSTN781 as an immunogen. Furthermore, 13 hybridoma clones (#6-1-2, #6-3-1, #6-9-1, #6-10-2, #6-13-2, #6-14-3, #6-16-3, #6-19-1, #6-20-1, #6-23-5, #6-43-1, #6-45-1 and #6-88-1) were obtained with the human periostin recombinant protein POSTN630 as an immunogen.

### (6) Reactivity of Anti-human Periostin Monoclonal Antibody

Reactivity on human periostin, of the anti-human periostin monoclonal antibody obtained in the section (5) above was verified as follows. The monoclonal antibodies were purified from the culture supernatant of each of hybridoma clones by a commonly-used affinity purification method with protein A Sepharose. The resulting purified antibodies were serially diluted with the highest concentration of 5µg/mL, and a reaction intensity against the human periostin recombinant protein POSTN781 or POSTN630 as an each immunogen was confirmed by the ELISA method.

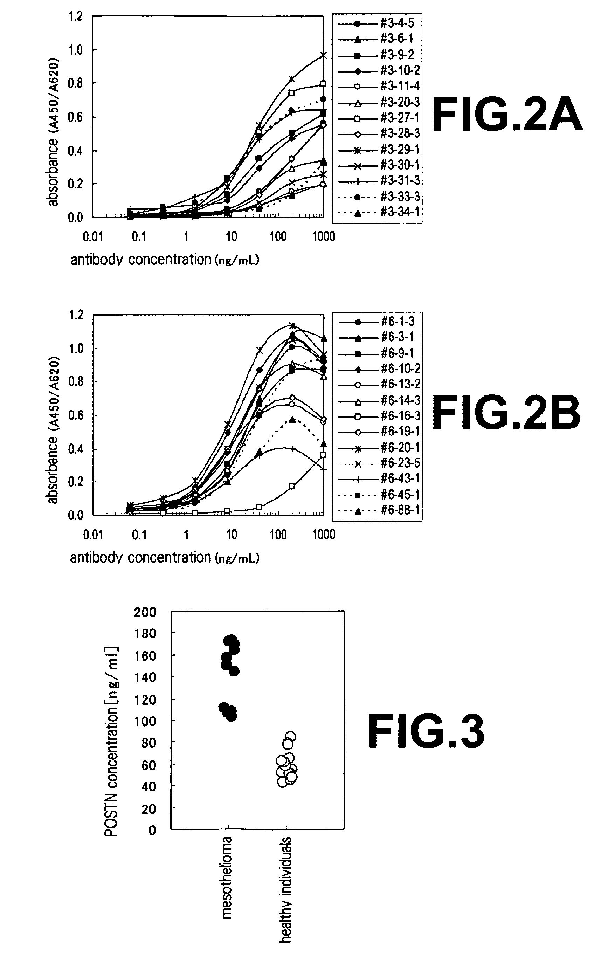

Fig. 2A is a graph showing results of reactivity of monoclonal antibodies raised against a human periostin recombinant protein POSTN781 as an immunogen by using a microplate coated with 500ng/mL POSTN781. Fig. 2B is a graph showing results of reactivity of monoclonal antibodies raised against a human periostin recombinant protein POSTN630 as an immunogen by using a microplate coated with 500 ng/mL POSTN630. As shown in Figs. 2A and B, it was confirmed that all monoclonal antibody clones reacted to the recombinant proteins used as the immunogen in a concentration dependent manner. On the other hand, when other recombinant human proteins having myc tag and his tag, which are identical to those of the human periostin recombinant protein, was tested similarly by the ELISA method, it did not react with the antibody clones. Thus, it was confirmed that any of the monoclonal antibody clones obtained according to the section (5) did not recognize an oligopeptide protion of the tag sequence. In addition, all 13 monoclonal antibody clones raised against POSTN781 as an immunogen reacted to POSTN630 as well. Accordingly, it was concluded that all anti-human periostin monoclonal antibody clones obtained in this Example recognized a polypeptide having 630 amino acid residues (SEQ ID NO: 2) that are common to all isoforms of the human periostin protein covering four fasciclin domains.

### (7) Establishment of Sandwich ELISA Method

In order to use as a detection antibody for a sandwich ELISA method, all monoclonal antibodies obtained in this Example were labelled with biotin. More specifically, an antibody solution was mixed with 10 mM EZ-Link Sulfo-NHS-LC-Biotin (Thermo, Funakoshi Co., Ltd.) in a volume of 16.7 µL per mg of the antibody, and the mixture was incubated at room temperature for 1 hour, followed by dialysis against PBS to eliminate unlabelled biotin. From among 26 monoclonal antibody clones, a combination of antibody clones suited for capture and detection in the sandwich ELISA method was determined by examining all candidate combinations of 26 x 26 as in the following. First, each 50 µL of a solution of 26 antibody clones having a concentration of 10 µg/mL prepared by diluting with a solution of 0.1 M NaHCO₃, 0.1 M Na₂CO₃ and 0.15% Proclin 150 (SUPELCO, Sigma-Aldrich Japan K.K.) was added to each well of a 96-well microplate, and left to stand at 4°C overnight or at room temperature for 2 hours to immobilize the antibody onto the bottom face of each well of the microplate. After the solution was eliminated, each 150 µL of a blocking buffer (PBS supplemented with 1% BSA (Proliant), 0.15% Proclin 150 and 5% sucrose) was dispensed, followed by leaving to stand at 4°C overnight or at room temperature for 2 hours. After the blocking buffer was eliminated, 50 µL of a solution of POSTN781 protein having a concentration of 10 ng/mL, 1 ng/mL or 0 ng/mL prepared by diluting with PBS supplemented with 1% BSA, 0.1% Tween20, 0.15% Proclin 150 and 50 µg/mL MAK-33 (Roche Diagnostics K.K.) was dispensed, and left to stand at room temperature for 1 hour. After the POSTN781 protein solution was eliminated, washing with PBS-0.05% Tween20 was conducted three times. Each 50µL of a solution of 26 antibody clones labelled with biotin was dispensed at a concentration of 5µg/mL, and left to stand at room temperature for 1 hour. After the solution of the biotin-labelled antibody clone was eliminated, washing with PBS-0.05% Tween20 was conducted three times. Each 50 µL of a solution of Streptavidin-poly HRP40 (SDT, Funakoshi Co., Ltd.) prepared by diluting 20, 000 fold with 1% BSA, 0.135 M NaCl, 0.1% p-Hydroxyphenylace, 0.15% Proclin 150, 10 mg/L bromophenol blue and 20 mM HEPES was dispensed, and left to stand at room temperature for 1 hour. After the solution of Streptavidin-polyHRP40 was eliminated, washing with PBS-0.05% Tween20 was conducted three times. Each 50 µL of TMB-US (Moss, Cosmo Bio Company, Limited) was dispensed and left to stand at room temperature for 30 min to permit color development, and thereafter 50 µL of 0.18 M H₂SO₄ was dispensed to stop the color development. The absorbance A450/A620 was measured using a spectrophotometer. Combinations of antibodies leading to the difference between the absorbance in 10 ng/mL POSTN781 protein well and that in 0 ng/mL POSTN781 protein well being no less than 2.0, and also leading to the difference between the absorbance in the well containing 1 ng/mL antigen and that in the well containing 0 ng/mL antigen being no less than 1.0 were selected. Combinations of selected monoclonal antibodies are as shown in Table 1 below.

**Table 1**

| Immobilized antibody | Detection antibody |
|---|---|
| #3-6-1 | #3-34-1 |
| #3-9-2 | #6-19-1 |
| #3-11-4 | #3-34-1 |
| #3-30-1 | #3-34-1 |
| #3-31-3 | #3-34-1 |
| #3-34-1 | #6-23-5 |
| #6-1-2 | #3-34-1 |
| #6-13-2 | #6-19-1 |
| #6-14-3 | #3-34-1 |
| #6-19-1 | #3-34-1 |
| #6-20-1 | #3-34-1 |
| #6-23-5 | #3-34-1 |
| #6-23-5 | #6-19-1 |
| #6-88-1 | #3-34-1 |
| #6-88-1 | #6-3-1 |
| #6-88-1 | #6-23-5 |

### Example 2

### Measurement of Clinical Specimen

### (1) Reactivity to Clinical Specimen

With regard to the specimens from healthy individuals, an approval by Ethics Committee Organization of Medical & Biological Laboratories Co., Ltd.; MBL ethics review board (Project number: 022; Date of Approval: March 27, 2007) was obtained and then specimens from healthy volunteers were solicited. Written consent for the measurement was obtained beforehand from each healthy individual who donated the specimen. With regard to specimens from mesothelioma patients, the aforementioned plasma specimens diluted 1,000 fold were used in place of human periostin purified recombinant proteins for the sandwich ELISA method explained in Example 1, section (7) which had been requested and obtained from BMR (Bio Medical Resources (integrated with Sera Care Life Sciences Milford, MA., at the time of application)). Results of measurements in connection with the combinations of antibodies shown in Table 1 on the plasma specimens from 16 healthy individuals, and plasma specimens from 11 mesothelioma patients are shown in Table 2.

**Table 2**

| Combination (immobilized antibody/detection antibody) | Mesothelioma specimen | Healthy persons specimen | p-value (t-test) |
|---|---|---|---|
| #3-6-1/#3-34-1 | 0.987 ±0.236 | 0.603 ±0.110 | 0.0002742 |
| #3-9-2/#6-19-1 | 0.267 ±0.028 | 0.193 ±0.027 | 0.0000007 |
| #3-11-4/#3-34-1 | 1.506 ±0.146 | 1.173 ±0.182 | 0.0000035 |
| #3-30-1/#3-34-1 | 1.281 ±0.158 | 1.004 ±0.145 | 0.0000998 |
| #3-31-3/#3-34-1 | 1.396 ±0.159 | 1.002 ±0.114 | 0.0000034 |
| #3-34-1/#6-23-5 | 0.871 ±0.150 | 0.606 ±0.076 | 0.0001461 |
| #6-1-2/#3-34-1 | 0.842 ±0.159 | 0.511 ±0.107 | 0.0000203 |
| #6-13-2/#6-19-1 | 0.739 ±0.132 | 0.514 ±0.156 | 0.0002730 |
| #6-14-3/#3-34-1 | 0.935 ±0.154 | 0.576 ±0.128 | 0.0000054 |
| #6-19-1/#3-34-1 | 0.505 ±0.085 | 0.340 ±0.055 | 0.0000540 |
| #6-20-1/#3-34-1 | 0.987 ±0.155 | 0.648 ±0.082 | 0.0000148 |
| #6-23-5/03-34-1 | 0.998 ±0.151 | 0.537 ±0.108 | 0.0000003 |
| #6-23-5/#6-19-1 | 0.808 ±0.121 | 0.593 ±0.128 | 0.0001311 |
| #6-88-1/#3-34-1 | 0.673 ±0.142 | 0.418 ±0.068 | 0.0001237 |
| #6-88-1/#6-3-1 | 0.835 ±0.162 | 0.463 ±0.182 | 0.0000060 |
| #6-88-1/#6-23-5 | 1.325 ±0.209 | 0.847 ±0.246 | 0.0000070 |

As shown in Table 2, the plasma specimens from the mesothelioma patients exhibited significantly greater measurements in all combinations, as compared with the plasma specimens from the healthy persons. In addition, a t-test revealed a p-value of less than 0.0005 in all cases. From these results, a possibility that the blood level of the periostin protein can serve as a marker for diagnosing mesothelioma was suggested.

### (2) Establishment of Sandwich ELISA Method for Measuring human Periostin Blood Level

Before examining in more detail on availability of the human periostin blood level as a marker for diagnosing mesothelioma using various clinical specimens, best suited combination of the immobilized antibody and the detection antibody was selected from the list shown in Table 1. Reactivity was evaluated to a series of two-fold dilution at seven different concentrations up to 2ng/mL, prepared with POSTN781 as a standard substance and a diluent not containing any POSTN781. Thus, as a typical example of the combination resulting in high reactivity to the standard substance and low absorbance of the blank, a combination of the immobilized antibody of #6-14-3, and the detection antibody of #3-34-1 was selected. In any of the following experiments, this combination of antibodies was employed.

### Example 3

### Measurement of Clinical Specimens

### (1) Measurement of Concentration of Periostin in Plasma Samples of Mesothelioma Patients and Healthy Individuals

Each 50 µL of the anti-human periostin monoclonal antibody #6-14-3 diluted to 10 µg/mL with a solution of 0.1 M NaHCO₃, 0.1 M Na₂CO₃ and 0.15% Proclin 150 (SUPELCO) was coated on a MaxiSorp 96-well plate (NUNC, Thermo Fisher Scientific K.K.), and left to stand at 4°C overnight or at room temperature for 2 hours to allow the antibody to be immobilized. After the solution of the antibody was eliminated, each 150 µL of a blocking buffer (PBS supplemented with 1% BSA (Proliant, Veritas Corporation), 0.15% Proclin 150 and 5% sucrose) was dispensed, followed by leaving to stand at 4°C overnight or at room temperature for 2 hours. After the blocking buffer solution was eliminated, using PBS supplemented with 1% BSA, 0.1% Tween20, 0.15% Proclin 150 and 50 µg/mL MAK-33 as a dilution liquid, a periostin standard substance (a series of dilution prepared by 2-fold and 7-times dilution of POSTN781 of from 2 µg/mL), a 500-fold dilution liquid of the plasma specimen (from 11 mesothelioma patients (BMR), and 16 healthy individuals), and the dilution liquid alone containing neither POSTN781 nor the plasma specimen were prepared, and each 50 µL was dispensed into each well. After leaving to stand at room temperature for 1 hour, solutions such as the dilution liquid for plasma specimens were eliminated, and washing with PBS-0.05% Tween20 was conducted three times. Biotinized #3-34-1 at a concentration of 1 µg/mL was dispensed in a volume of 50 µL. After leaving to stand at room temperature for 1 hour, the solution of biotinized #3-34-1 was eliminated, and washing with PBS-0.05% Tween20 was conducted three times. Each 50 µL of a solution of Streptoavidin-poly40HRP (SDT) prepared by diluting 20,000 fold with a dilution liquid containing 1% BSA, 0.135 M NaCl, 0.1% p-Hydroxyphenylace, 0.15% Proclin 150, 10 mg/L bromophenol blue and 20 mM HEPES was dispensed, and left to stand at room temperature for 1 hour. After the solution of Streptoavidin-poly40HRP was eliminated, washing with PBS-0.05% Tween20 was conducted three times. Each 50 µL of TMB-US (Moss, INC.) was dispensed and left to stand at room temperature for 30 min to permit color development, and thereafter 50 µL of 0.18 M H₂SO₄ was dispensed to stop the color development. The absorbance A450/A620 was measured using a spectrophotometer, and the concentration of periostin contained in the specimen was quantitatively determined from the absorbance. Fig. 3 shows a graph illustrating distributions of concentrations of periostin in specimens from mesothelioma patients and healthy individuals, respectively. Regarding concentration of periostin in the specimens from the mesothelioma patients and healthy individuals, a p-value for a t-test was calculated, and turned out to be 0.00000066 indicating the significant statistical differences between them.

### (2) Correlation with Measurements of Soluble Mesothelin Related Protein (SMRP)

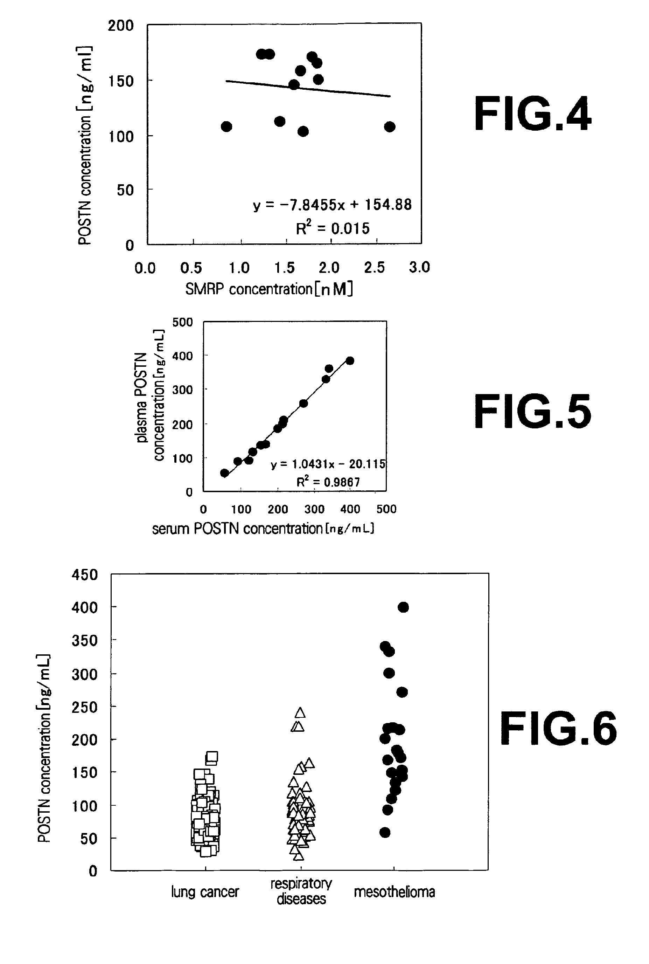

In the plasma specimens of which concentration of periostin was measured as in the above section (1) (11 mesothelioma patients (BMR), and 16 healthy individuals), concentration of a soluble mesothelin related protein (SMRP) that is a conventionally used marker for diagnosis of mesothelioma in serum was measured in accordance with a manufacturer's instruction of a kit for measuring the concentration of SMRP (MESOMARK, Fujirebio Diagnostic). Fig. 4 shows a correlation diagram obtained by plotting concentrations of periostin and SMRP in plasma identical specimens from the mesothelioma patients. As shown in Fig. 4, no correlation was found between the measurements of concentrations of periostin and SMRP in plasma. When a cut-off value of periostin is tentatively postulated to be 100 ng/mL, all 11 cases were found to be positive, whereas only 7 cases (64%) were determined to be positive for SMRP when a cut-off value of 1.5nM as a reference value was adopted. It was also revealed that all four cases (36%) indicating a negative result for SMRP exhibited a positive result for periostin.

### Example 4

### Analyses of Specimens from Patients Collected by Respiratory Division Group, Graduate School of Medicine, Nagoya University

The experiments of this Example were carried out under an approval by bioethics review board, Gaduate School of Medicine, Nagoya University (Approval Number: 546-4, Date of Approval: July 29, 2011). Written consent was obtained from patients who donated the specimens.

The concentrations of periostin were measured in the plasma and serum specimens collected from the patients by Respiratory Division Group, Graduate School of Medicine, Nagoya University. The malignant pleural mesothelioma patients who donated the specimens suffered from malignant pleural mesothelioma of epithelial type, biphasic type and sarcomatoid type, and the numbers of those patients were 14, 5 and 2, respectively. The determination on the type of the malignant pleural mesothelioma, as to which of the epithelial type, biphasic type or sarcomatoid type was made in accordance with Husain et al., (Arch. Pathol. Lab. Med., 133: 1317, 2009), using a haematoxylin-eosin staining method. 118 lung cancer patients, and 53 patients suffering from a respiratory organ disease other than cancer such as, e.g., COPD were involved.

First, according to a routine method, concentrations of periostin in the plasma and serum specimens obtained from the same patient were measured by the same sandwich ELISA method as that of Example 3. Fig. 5 shows a graph demonstrating comparison of concentrations of periostin in the plasma and serum specimens. As shown in Fig. 5, the concentration of periostin in the serum and plasma exhibited approximately the same measurements in the same patients. Therefore, it was ascertained that concentrations of periostin did not differ between the plasma and serum specimens from the same patient. Accordingly, the serum specimen was used in the following Examples for the measurement of the concentration of periostin.

Fig. 6 shows a graph demonstrating of concentrations of periostin in the serum specimens obtained from patients of malignant pleural mesothelioma, lung cancer, and various respiratory diseases other than cancer (represented by "respiratory diseases" in Fig. 6). As is clear from Fig. 6, the concentrations of periostin in specimens obtained from malignant pleural mesothelioma patients were higher than those from other patients.

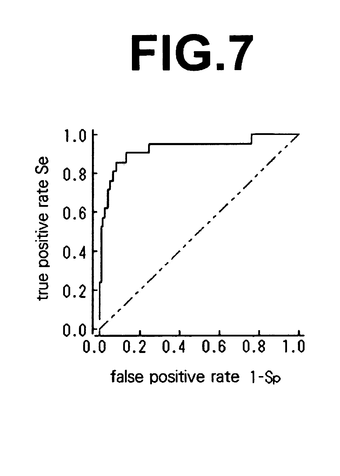

Fig. 7 shows a graph illustrating an ROC curve drawn using the concentrations of periostin as a marker of malignant pleural mesothelioma patients of this Example with respect to patients suffering from various respiratory diseases other than mesothelioma including lung cancer and a respiratory organ disease other than cancer in combination. As a result of ROC analysis of this Example, AUC was revealed to be 0.927. When the accuracy of diagnosis was calculated with a tentative cut-off value of the concentration of periostin being postulated to be 133ng/mL, sensitivity to malignant pleural mesothelioma patients was 0.810. In addition, specificity for the patient suffering from various respiratory diseases other than malignant pleural mesothelioma was 0.936. Accordingly, it was also indicated that periostin can be a very effective marker also in distinguishing malignant pleural mesothelioma patients from patients suffering from a respiratory organ disease other than malignant pleural mesothelioma.

When the specimens from malignant pleural mesothelioma patients were analyzed on each tissue type with the tentative cut-off value of the concentration of periostin being postulated to be 133 ng/mL, positive detection was found in 4 of 5 specimens (80%) obtained from patients of biphasic malignant pleural mesothelioma, whereas positive detection was found in 2 of 2 specimens (100%) obtained from patients of sarcomatoid malignant pleural mesothelioma. On the other hand, when SMRP was employed as test of malignant pulmonary mesothelioma, positive detection was found in 1 of 5 specimens (25%) obtained from biphasic malignant pleural mesothelioma patients, whereas no serum specimens (0%) obtained from two sarcomatoid malignant pleural mesothelioma patients was positive for detection. Accordingly, it was proven that detection of an increase in the concentration of periostin in the blood is useful for a marker for diagnosis of biphasic and sarcomatoid malignant pulmonary mesothelioma, which are more aggressive types of mesothelioma.

### Example 5

### Periostin Secreted by Established Cancer Cells

In order to study with the sandwich ELISA method of the present invention, established cancer cell lines shown in Table 3 were used to evaluate how much periostin is secreted into a medium from various types of cancer cells.

**Table 3**

| Cancer type | Strain name of Established cancer cell | Code No. |
|---|---|---|
| lung cancer | A431 | ATCC; CRL-1555 |
| lung cancer | A549 | ATCC; CCL-185 |
| lung cancer | ChaGo-K-1 | ATCC; HTB-168 |
| lung cancer | DMS 144 | ATCC; CRL-2066 |
| lung cancer | NCI-H358 | ATCC; CRL-5807 |
| lung cancer | NCI-H441 | ATCC; HTB-174 |
| lung cancer | NCI-H460 | ATCC; HTB-177 |
| lung cancer | NCI-H522 | ATCC; CRL-5810 |
| lung cancer | NCI-H596 | ATCC; HTB-178 |
| lung cancer | NCI-H1299 | ATCC; CRL-5803 |
| lung cancer | NCI-H1373 | ATCC; CRL-5866 |
| lung cancer | NCI-H1395 | ATCC; CRL-5868 |
| lung cancer | NCI-H1793 | ATCC; CRL-5896 |
| lung cancer | NCI-H1975 | ATCC; CRL-5908 |
| lung cancer | NCI-H2170 | ATCC; CRL-5928 |
| lung cancer | PC-14 | ECACC; EC90071810 |
| lung cancer | SK-LU-1 | ATCC; HTB-57 |
| lung cancer | SK-MES-1 | ATCC; HTB-58 |
| mesothelioma | NCI-H28 | ATCC; CRL-5820 |
| mesothelioma | NCI-H226 | ATCC; CRL-5826 |
| mesothelioma | NCI-H2052 | ATCC; CRL-5915 |
| mesothelioma | NCI-H2452 | ATCC; CRL-5946 |
| mesothelioma | MSTO | ATCC; CRL-2081 |
| colon cancer | HT-29 | ATCC; HTB-38 |
| colon cancer | LoVo | ATCC; CCL-229 |
| colon cancer | SW480 | ATCC; CCL-228 |
| colon cancer | SW620 | ATCC; CCL-227 |
| kidney cancer | ACHN | ATCC; CRL-1611 |
| kidney cancer | Caki-1 | ATCC; HTB-46 |
| kidney cancer | G401 | ATCC; CRL-1441 |
| stomach cancer | GCIY | BRC; RCB0555 |
| stomach cancer | KATO III | ATCC; HTB-103 |
| stomach cancer | MKN1 | BRC; RCB1003 |
| stomach cancer | MKN45 | BRC; RCB1001 |
| stomach cancer | MKN74 | BRC; RCB1002 |
| stomach cancer | SCH | HSRRB; JCRB0251 |
| pancreatic cancer | KLM-1 | BRC; RCB2138 |
| pancreatic cancer | MIA PaCa-2 | ATCC; CRL-1420 |
| pancreatic cancer | PANC-1 | ATCC; CRL-1469 |
| pancreatic cancer | PK-1 | BRC; RCB1972 |
| pancreatic cancer | PK-45P | BRC; RCB2141 |
| pancreatic cancer | PK-59 | BRC; RCB1901 |
| Hepatic cancer | Hep G2 | BRC; RCB1886 |
| breast cancer | BT-20 | ATCC; HTB-19 |
| breast cancer | BT-474 | ATCC; HTB-20 |
| breast cancer | BT-549 | ATCC; HTB-122 |
| breast cancer | HCC-1395 | ATCC; CRL-2324 |
| breast cancer | HCC-1937 | ATCC; CRL-2336 |
| breast cancer | MCF7 | ATCC; HTB-22 |
| breast cancer | MDA-MB-231 | ATCC; HTB-26 |
| breast cancer | SK-BR-3 | ATCC; HTB-30 |
| breast cancer | T-47D | ATCC; HTB-133 |
| breast cancer | ZR-75-1 | ATCC; CRL-1500 |
| prostate | 22Rv1 | ATCC; CRL-2505 |
| prostate cancer | PC-3 | ATCC; CRL-1435 |
| bladder cancer | T24 | ATCC; HTB-4 |
| uterine cervical cancer | HeLa | ATCC; CCL-2 |
| uterine cervical cancer | SiHa | ATCC; HTB-35 |
| ovary adenoma | SK-OV-3 | ATCC; HTB-77 |
| glioma | T98G | ATCC; CRL-1690 |
| Burkitt lymphoma | Daudi | ATCC; CCL-213 |
| Burkitt lymphoma | Raji | ATCC; CCL-86 |
| leukemia, acute T lymphoblastic | CCRF-CEM | ATCC; CCL-119 |
| T cellular leukemia | Jurkat | ATCC; TIB-152 |
| T cell leukemia | MOLT-4 | BRC; RCB0206 |
| T cellular leukemia | PEER | BRC; RCB1879 |
| promyelocytic leukemia | HL-60 | ATCC; CCL-240 |
| monocytic leukemia | THP-1 | ATCC; TIB-202 |
| lymphoma-derived monoblastoid cell | U-937 | ATCC; CRL-1593.2 |
| erythrocytic leukemia | HEL | ATCC; TIB-180 |
| megakaryoblastic leukemia | MEG-01 | ATCC; CRL-2021 |
| umbilical cord-derived normal vascular endothelial cell | HUVEC | ATCC; CRL-2873 |

Abbreviations of Code Nos. in Tables are explained as in the following.
ATCC: American Type Culture Collection
BRC: RIKEN BioResource Center
HSRRB: Health Science Research Resources Bank

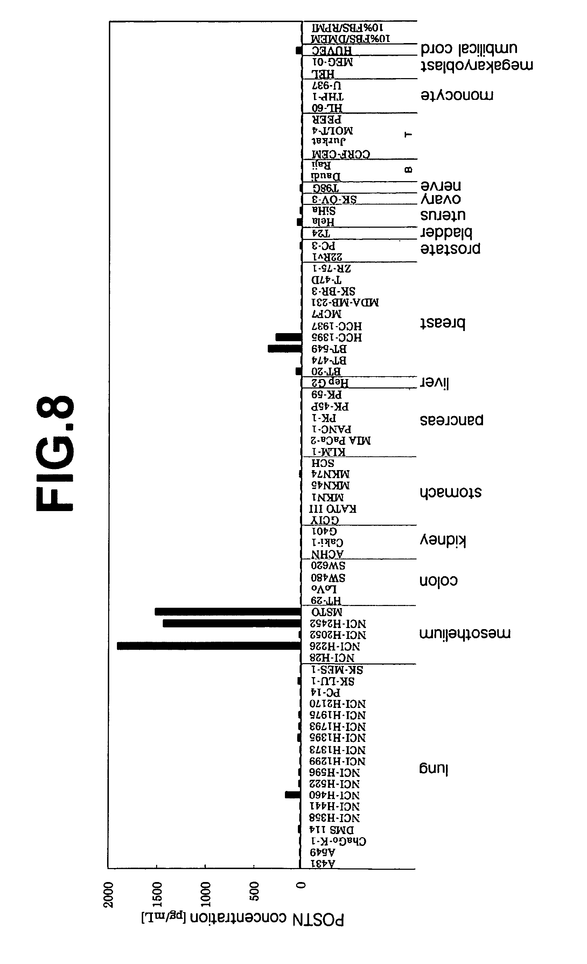

Fig. 8 shows a graph demonstrating results obtained by measuring periostin in culture supernatants of established cancer cells shown in Table 2 using a measurement system involving the immobilized antibody of #6-14-3 and the detection antibody of #3-34-1. In cancer cell strains derived from mesothelioma, a high concentration of periostin was detected in 3 types among 5 types (60%) (Fig. 9). On the other hand, in other cases, some extent of expression was found in a part of lung cancer and breast cancer cases; however, the amount of expression was less than quarter, and no expression was observed in the cases of colon cancer, kidney cancer, stomach cancer, pancreatic cancer, liver cancer, prostate gland cancer, bladder cancer, uterus cancer, ovary cancer, neuroblastoma, B cellular and T cellular, monocytic, megakaryoblastic blood cancer, and umbilical cord blood-derived vascular endothelial cells. Thus, it was proven that the expression and the presence in the culture media of periostin are considerably specific for cancer cell strains derived from mesothelioma.

### Example 6

### Measurement of Pleural Fluid Specimen

In order to study as to whether or not pleural fluid samples can be used as a clinical specimen in addition to the plasma samples and serum samples, concentrations of periostin in pleural fluid samples from one mesothelioma patient, and as a control from one lung adenocarcinoma patient were measured. The experiments of this Example were carried out also under an approval by Nagoya University, School of Medicine, bioethics review board (Approval number: 546-5, Date of Approval: March 27, 2012). Written consent was obtained from patients who donated the specimens.

The malignant pleural mesothelioma patient who donated the specimens suffered from biphasic malignant pleural mesothelioma. According to a routine method, concentrations of periostin in the plasma specimen, serum specimen and pleural fluid specimen prepared from the same patient were measured by the same sandwich ELISA method as those of Examples 3 and 4. The collected pleural fluid specimens were prepared by the same method as those of plasma specimens and serum specimens. Since the concentration of periostin in the pleural fluid specimen was higher than those of the serum specimen and plasma specimen, a 16,000-fold diluted liquid was subjected to binding to the immobilized antibody.

As a result, the concentrations of periostin in the plasma specimen, serum specimen and pleural fluid specimen from the mesothelioma patient were 247.9ng/mL, 279.2ng/mL and 6928.7ng/mL, respectively. The concentrations of periostin in the plasma specimen, serum specimen and pleural fluid specimen from the lung adenocarcinoma patient measured concomitantly were 47.4ng/mL, 53.6ng/mL and 1938.4ng/mL, respectively. The concentration of periostin in the serum specimen from the mesothelioma patient exceeded the tentative cut-off value of the concentration of periostin in the serum specimen being 133 ng/mL defined in Example 4, indicating a positive diagnosis based on the concentration of periostin in the serum specimen. The concentrations of periostin in the pleural fluid specimens were several ten-fold higher both in the mesothelioma patient and in the lung adenocarcinoma patient, as compared with the concentrations of periostin in the plasma specimen and serum specimen. Additionally, also in the pleural fluid specimen, the concentration of periostin in the mesothelioma patient was 3 or more-fold greater as compared with that in the lung adenocarcinoma patient. Therefore, it was suggested that the method for testing mesothelioma carried out using the concentration of periostin of the present invention can be applied also to pleural fluid specimens.

### INDUSTRIAL APPLICABILITY

As explained hereinabove, any malignant pleural mesothelioma of epithelial types, biphasic type and sarcomatoid type can be detected according to the method for testing mesothelioma of the present invention. Accordingly, early diagnoses of mesothelioma are enabled without imposing a heavy burden such as biopsy onto patients; therefore, significant contributions to early treatments of mesothelioma patients can be made. In addition, over several million people who exposed to asbestos can be provided with a highly reliable, simple and inexpensive testing method in screening mesothelioma; therefore, those exposed to asbestos can be easily and repeatedly subjected to testing. Thus, early detection and treatment of mesothelioma patients are enabled, leading to great contributions to improvements of cure rates. Moreover, it becomes also possible to diagnose mesothelioma from pleural fluids collected from patients showing symptoms resulting from pleural effusion.

## Claims

1. A method for testing mesothelioma, comprising:
a step of determining a concentration of a human periostin protein in at least one type of samples of blood and pleural fluid of a subject.

2. The method for testing mesothelioma according to claim 1, wherein an antibody directed against a human periostin protein is used in the step of determining the concentration of the human periostin protein.

3. The method for testing mesothelioma according to claim 1 or 2, wherein the antibody directed against a human periostin protein is an antibody that binds to a polypeptide consisting of an amino acid sequence set out in SEQ ID NO: 2.

4. The method for testing mesothelioma according to any one of claims 1 to 3, wherein two types of antibodies capable of concurrently binding to the human periostin protein are used in the step of determining the concentration of the human periostin protein.

5. The method for testing mesothelioma according to claim 4, wherein the mesothelioma is malignant pleural mesothelioma.

6. The method for testing mesothelioma according to claim 4, wherein the mesothelioma is sarcomatoid malignant pleural mesothelioma or biphasic malignant pleural mesothelioma.

7. The method for testing mesothelioma according to any one of claims 1 to 6, wherein the blood sample is a plasma sample or a serum sample from the subject.

8. A kit for diagnosing mesothelioma, comprising an antibody directed against a human periostin protein.

9. The kit for diagnosing mesothelioma according to claim 8, wherein the antibody directed against a human periostin protein is an antibody that binds to a polypeptide consisting of an amino acid sequence set out in SEQ ID NO: 2.

10. The kit according to claim 9, wherein the antibody directed against a human periostin protein is two types of antibodies capable of concurrently binding to the human periostin protein.

11. The kit for diagnosing mesothelioma according to claim 10, comprising:
a solid support on which one of the two types of antibodies capable of concurrently binding to the human periostin protein is immobilized; and
a specific binding partner for another of the two types of antibodies.

12. The kit for diagnosing mesothelioma according to any one of claims 8 to 11, wherein diagnosing mesothelioma is to determine as to whether or not a subject who may be suffering from mesothelioma is a mesothelioma patient.

13. The kit for diagnosing mesothelioma according to any one of claims 8 to 11, wherein diagnosing mesothelioma is to determine as to whether a subject who may be suffering from mesothelioma is a mesothelioma patient, or a patient suffering from a respiratory organ disease other than mesothelioma.

14. The kit for diagnosing mesothelioma according to claims 12 or 13, wherein the mesothelioma patient is a malignant pleural mesothelioma patient.

15. The kit for diagnosing mesothelioma according to claim 14, wherein the mesothelioma patient is a sarcomatoid malignant pleural mesothelioma patient or a biphasic malignant pleural mesothelioma patient.
